# EUROPEAN PATENT APPLICATION

(11) **EP 2 730 257 A1**
(43) Date of publication of application: **14.05.2014**
(21) Application number: 12192014.4
(22) Date of filing: 09.11.2012
(51) Int. Cl.: A61F 5/44, D04B 1/24, D04B 1/26

(54) **A method for providing a weft-knitted fixation garment**

(71) Applicant: TYTEX A/S, 7430 Ikast (DK)
(72) Inventor: Karlsen, Kresten, 8600 Silkeborg (DK); Özdemir, Cengiz, 7430 Ikast (DK); Rasmussen, Knud Fjelsted, 7430 Ikast (DK)
(74) Representative: Hoffmann Dragsted A/S

(57) **Abstract**

The present invention relates to a method for providing a weft-knitted fixation garment to be worn on a wearer's arm or leg. The fixation garment comprises a knitted tubular part having a circumference less than 1000 mm, preferably less than 500 mm, the knitted tubular part having a first end and a second end and comprising a first layer and a second layer extending therebetween, the weft-knitted fixation garment comprising a first side and a second side and a pocket adapted to fixate an object to the wearer's arm or leg arranged at the first side. The fixation garment is integrally knitted in one process on a circular knitting machine.

## Description

### Field of the invention

The present invention relates to a method for providing a weft-knitted fixation garment to be worn on a wearer's arm or leg, the fixation garment comprising a knitted tubular part having a circumference less than 1000 mm, preferably less than 500 mm, the knitted tubular part having a first end and a second end and comprising a first layer and a second layer extending there between, the weft-knitted fixation garment comprising a first side and a second side and a pocket adapted to fixate an object to the wearer's arm or leg arranged at the first side.

Furthermore, the present invention relates to a weft-knitted fixation garment for fixing an object to an arm or leg of a wearer and to use of said weft-knitted fixation garment for fixing a urine bag, an ice pack, an electrical device or similar objects to an arm or leg of a wearer.

### Background art

Fixation garments for assisting in fixating objects to an arm or a leg of a user are known, cf. WO9633675A1 which discloses a garment for instance for fixing a urine bag to the leg of a user. Even though the garment disclosed in WO9633675A1 is provided by knitting on a double Raschel knitting machine, it requires subsequent tailoring before it is ready to be used. This is a cumbersome process since the manufacturing of a fixation garment involves several steps.

### Summary of the invention

It is an object of the present invention to wholly or partly overcome the above disadvantages and drawbacks of the prior art. More specifically, it is an object to provide an improved method for providing a fixation garment.

Moreover, it is an object of the present invention to provide a method for providing a fixation garment which may be manufactured in one step without subsequent tailoring.

It is also an object to provide a fixation garment having enhanced support properties for the object to be fixated.

The above objects, together with numerous other objects, advantages, and features, which will become evident from the below description, are accomplished by a solution in accordance with the present invention by a method for providing a weft-knitted fixation garment to be worn on a wearer's arm or leg, the fixation garment comprising a knitted tubular part having a circumference less than 1000 mm, preferably less than 500 mm, the knitted tubular part having a first end and a second end and comprising a first layer and a second layer extending therebetween, the weft-knitted fixation garment comprising a first side and a second side and a pocket adapted to fixate an object to the wearer's arm or leg arranged at the first side, wherein the fixation garment is integrally knitted in one process on a circular knitting machine by:
- knitting a first rib edge of the tubular part,
- knitting the first layer of the tubular part,
- knitting the second layer of the tubular part directly after the first layer,
- knitting a second rib edge of the tubular part, and
- inserting the second layer into the first layer for providing a seamless fixation garment ready for use.

The term "circumference" is, in this context, to be construed as being the extension or distance measured around or along the periphery of the fixation garment seen in a cross-sectional view. For instance, the circumference of a circle is nd, where n is phi and d is the diameter of the circle. However, a more practical manner of measuring the circumference of the fixation garment is to place the fixation garment on a flat surface and subsequently flatten the tube (while it is still in a relaxed state, i.e. without any tension), so that it becomes two-folded and essentially flat. In this way, it is possible to measure the width of the fabric and afterwards multiply the width by the factor 2, thus obtaining substantially the circumference of the fixation garment in question.

Moreover, the method may comprise the following steps:
- hanging up a plurality of stitches on transfer jacks or needles at a predetermined distance after the first rib edge, and
- knitting in the hanged-up plurality of stitches at a predetermined distance before a second rib edge so that the first and second layers of the tubular body are interknitted.

Furthermore, an aperture may be provided at the second end on the first side. The aperture may be provided by knitting in a water-soluble yarn or by leaving the needles empty of yarn during the knitting steps.

Additionally, the pocket may be provided between the first and second layers.

Also, an opening to the pocket may be provided at the first end between the first and second rib edges.

Moreover, the predetermined distance may be from the first rib edge to the start of the second layer, preferably between the first rib edge and half the distance to the second layer, and/or from the the second rib edge to the start of the first layer, preferably between the second rib edge and half the distance to the first layer.

Furthermore, the two layers may be knitted together along the entire circumference or part of the circumference of the tubular part.

Additionally, the tubular part may be knitted with different elasticity along a length of the tubular part and/or along the circumference of the tubular body.

Also, one or more fixation zones may be provided in the first layer, the fixation zones extending around the circumference of the first layer and being positioned at mutual distances in a length direction of the first layer.

Moreover, the fixation zones may be provided by knitting additional Elastane having a higher tension in the fixation zones, and/or changing the knitting structure in the fixation zones to a more elastic or stretchable structure than the adjacent layers.

Furthermore, the first layer may be knitted with an elasticity which is different from that of the second layer.

Additionally, the first layer may be knitted with a stretchability which is higher, at least on the first side of the tubular part, than on the second layer.

Also, the aperture may be provided in the first layer at a second distance from the second end.

Moreover, an intermediate area may be knitted along the circumference between the first and second layers with an elasticity which is higher than that of the adjacent first and second layers.

The invention also relates to a weft-knitted fixation garment for fixing an object to an arm or leg of a wearer provided by the method as described above. The fixation garment may comprise a knitted tubular part having a circumference less than 1000 mm, preferably less than 500 mm, the knitted tubular part having a first end and a second end and comprising a first layer and a second layer extending therebetween, the weft-knitted fixation garment comprising a first side and a second side and a pocket adapted to fixate an object to the wearer's arm or leg arranged at the first side.

Furthermore, the first side may comprise an aperture at the second end.

Additionally, an opening to the pocket may be arranged at the first end.

Also, the two layers may be knitted together at the second side at a predetermined distance from the first end.

Moreover, the predetermined distance may be from the first rib edge to the start of the second layer, preferably between the first rib edge and half the distance to the second layer, and/or from the the second rib edge to the start of the first layer, preferably between the second rib edge and half the distance to the first layer.

Furthermore, the two layers may be knitted together along the entire circumference or part of the circumference of the tubular part.

Additionally, the first layer may comprise one or more fixation zones, the fixation zones extending around the circumference of the first layer and being positioned at mutual distances in a length direction of the first layer.

Also, the fixation zones may have a higher elasticity than an adjacent area of the first layer.

Moreover, the tubular part may have a different elasticity along a length of the tubular part and/or along the circumference of the tubular body.

Furthermore, the first layer of the tubular part may have an elasticity which is different from that of the second layer of the tubular part.

Additionally, the first layer may have a stretchability which is higher, at least on the first side, than on the second side.

Also, the second layer may comprise skin-friendly yarns. Both the first and second layers may comprise yarns made of Elastane, polyester, polyamide, polypropylene, wool, cotton or viscose or a combination thereof.

Moreover, an aperture may be arranged in the first layer at a second distance from the second end.

Furthermore, the second end may have an intermediate area extending along the circumference between the first and second layers, the intermediate layer having a higher elasticity than the adjacent first and second layers.

The present invention also relates to a use of the weft-knitted fixation garment for fixing a urine bag, an ice pack, an electrical device or similar objects to an arm or leg of a wearer.

### Brief description of the drawings

The invention and its many advantages will be described in more detail below with reference to the accompanying schematic drawings, which for the purpose of illustration show some non-limiting embodiments and in which
Fig. 1 shows a knitted tubular part of the weft-knitted fixation garment according to the invention,
Fig. 2 shows a weft-knitted fixation garment in a folded position having the tubular part of Fig. 1 in a side view,
Fig. 3 shows another knitted tubular part,
Fig. 4 shows a weft-knitted fixation garment in a folded position having the tubular part of Fig. 3,
Fig. 5 shows another embodiment of a first side of a weft-knitted fixation garment in a folded position in a side view,
Fig. 6 shows a second side of the weft-knitted fixation garment of Fig. 5 in a folded position,
Fig. 7 shows the first layer folded back from the first end so that insertion of the object into the pocket is facilitated,
Fig. 8 shows the weft-knitted fixation garment of Fig. 5 with the first layer folded back at the second side seen from the first end, and
Fig. 9 shows a weft-knitted fixation garment positioned on a leg of a wearer.

All the figures are highly schematic and not necessarily to scale, and they show only those parts which are necessary in order to elucidate the invention, other parts being omitted or merely suggested.

### Detailed description of the invention

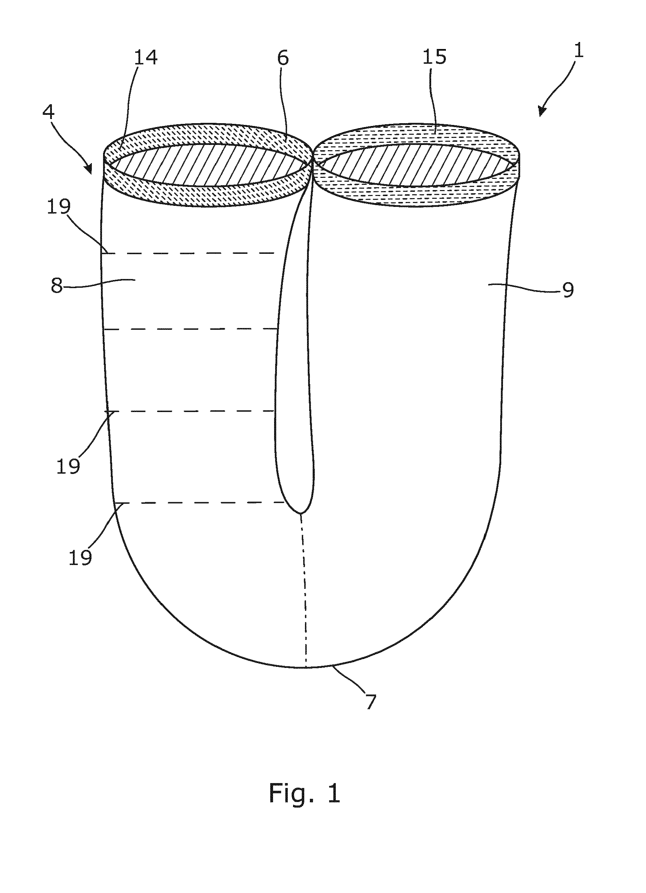

In Fig. 1, a weft-knitted fixation garment 1 according to the invention is shown after it has been integrally knitted in one step on a circular knitting machine. The fixation garment 1 comprises a knitted tubular part 4 having a circumference less than 1000 mm, preferably less than 500 mm. The knitted tubular part 4 has a first end 6 and a second end 7 and comprises a first layer 8 and a second layer 9 extending therebetween. The weft-knitted fixation garment 1 comprises a first side 10 and a second side (not shown) and a pocket (not shown) adapted to fixate an object to a wearer's arm or leg arranged at the first side 10.

As mentioned above, the weft-knitted fixation garment is knitted on a circular knitting machine, preferably for body size knitting, such as e.g. a Santoni knitting machine.

According to the inventive idea, the fixation garment 1 is integrally knitted in one process on a circular knitting machine by:
- knitting a first rib edge 14 of the tubular part 4,
- knitting the first layer 8 of the tubular part 4,
- knitting the second layer 9 of the tubular part directly after the first layer 8, and
- knitting a second rib edge 15 of the tubular part.

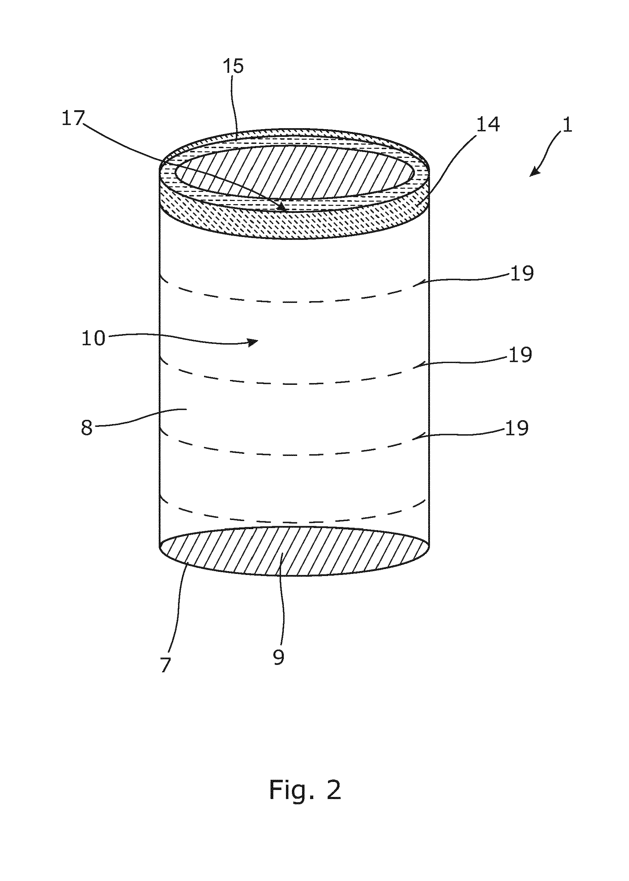

After the tubular part 4 has been knitted, the second layer 9 is inserted into the first layer 8 for providing a seamless fixation garment 1 ready for use, as shown in Fig. 2. The pocket is arranged between the first and second layers of the tubular part.

In this way, it is obtained that the fixation garment is knitted in one process only, and no subsequent tailoring is necessary. Thus, by means of the present invention it is possible to manufacture a high-end garment having several different properties and features incorporated, e.g. by combining materials, yarn types, knit with different tightness, different knitting structures, needle selection, knit-in text and logos, which - when it comes to manufacturing price - may be competitive compared to the prior art.

Furthermore, the first layer 8 comprises four fixation zones 19, the fixation zones 19 extending around the circumference of the first layer 8 and being positioned at mutual distances in a length direction of the first layer 8. The fixation zones 19 may be provided by knitting additional Elastane having a higher tension in the zones, and/or changing the knitting structure in the fixation zones to a more elastic or stretchable structure than the adjacent layer. In Fig. 1, the fixation zones 19 are shown as lines. However, in other embodiments the fixation zones may have other widths in relation of the intended use of the fixation garment.

Furthermore, an opening 17 to the pocket is provided at the first end 6 between the first and second rib edges 14, 15, as seen on Fig. 2. Thus, the opening 17 is formed by the double layer configuration of the tubular part 4.

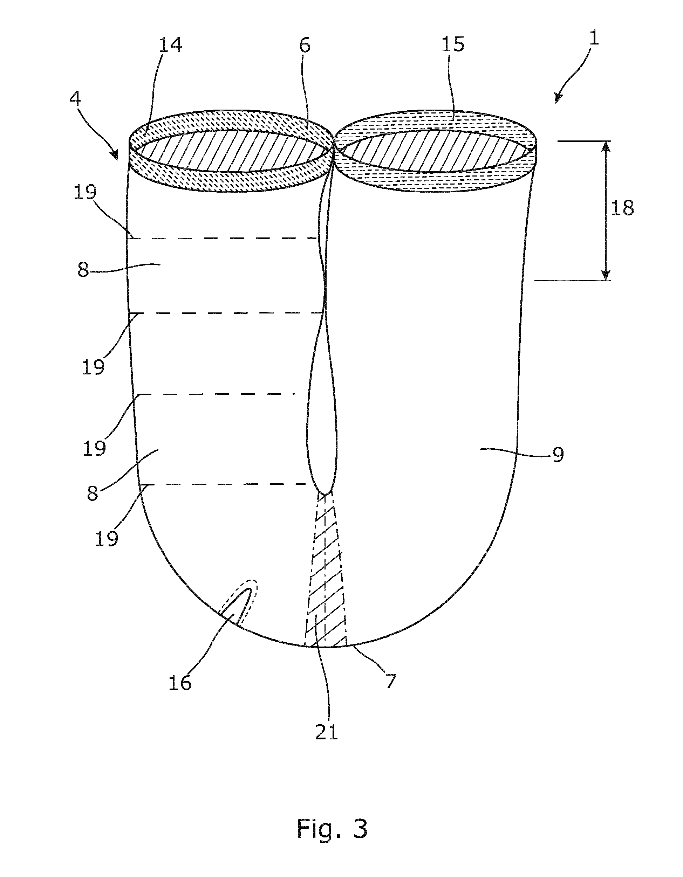

Fig. 3 shows a fixation garment 1 which is substantially identical to the embodiment shown in Figs. 1 and 2. However, in this embodiment the first and second layers have been interknitted before the second layer is inserted into the first layer.

This is performed by hanging up a plurality of stitches on transfer jacks or needles at a predetermined distance after the first rib edge 14, and knitting in the hanged-up plurality of stitches at a predetermined distance before a second rib edge 15 so that the first and second layers of the tubular body are interknitted.

By first knitting a tubular part 4 being interknitted and afterwards inserting the one layer into the other, it is obtained that the pocket is formed by the two layers, and the interknitted area is also part of defining the pocket, so that the object in the pocket is fixated.

In this embodiment, the first and second layers 8, 9 are interknitted at a predetermined distance 18 from the first end 6. The two layers 8, 9 are knitted together along the entire circumference or part of the circumference of the tubular part 4. In some embodiments, the two layers are knitted together along the circumference of the tubular part 4 in less than 50% of the circumference. In this way, it is obtained that the pocket is defined in a predetermined size, and that the pocket may be opened for facilitating insertion of the object into the pocket. This may for instance be performed by folding the first layer 8 downwards from the opening 17. In this embodiment, the first layer 8 may be folded downwards around the entire circumference up to the interknitted area.

In another embodiment, the first and second layers 8, 9 are interknitted in the vicinity of the rib edges 14, 15 so that the two layers are secured to each other at the first end 6.

In the same manner as in Fig. 1, the first layer 8 comprises four fixation zones 19, the fixation zones 19 extending around the circumference of the first layer 8 and being positioned at mutual distances in a length direction of the first layer 8. For instance, if the fixation garment 1 is adapted to fixate a urine bag, the fixation zones 19 ensure that the urine content in the urine bag will be distributed in the entire bag so that undesired outpouching of the bag is avoided. If the urine bag outpouches, it may be visible which is undesirable for the wearer of the fixation garment and the urine bag.

The fixation garment 1 comprises an aperture 16 at the second end 7 on the first side. The aperture 16 is provided by knitting in a water-soluble yarn or by leaving the needles empty of yarn. If water-soluble yarn is used, the aperture 16 will not be provided until after the fixation garment has been washed. The aperture 16 may be positioned in the opposite end of the opening to the pocket, and may provide access to the interior of the pocket. The aperture may for instance be used as a passage of a discharge pipe of the urine bag.

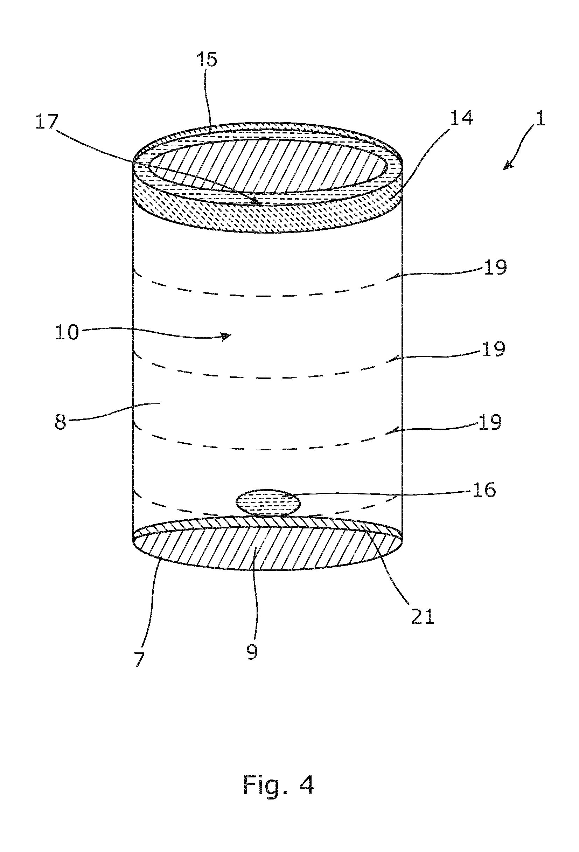

Furthermore, an intermediate area 21 is knitted along the circumference between the first and second layers 8, 9 with an elasticity which is different, preferably higher, than that of the adjacent first and second layers 8, 9. In this way it is obtained that the intermediate area 21 will have the same function as a rib edge when the second layer 9 has been inserted into the first layer 8, as shown in Fig. 4. The intermediate area 21 will assist in fixating the object inserted into the pocket. The intermediate area 21 may be knitted as a rib area, so that it may have the same appearance as the first end 6 of the fixation garment 1.

Furthermore, by means of the present invention it is possible to incorporate different properties in different areas and layers of the fixation garment 1. For instance, the tubular part may be knitted with different elasticity along a length of the tubular part and/or along the circumference of the tubular body. Additionally, the first layer may be knitted with an elasticity which is different from that of the second layer. Also, the first layer may be knitted with a stretchability which is higher, at least on the first side of the tubular part, than on the second side, so that the first side may expand as the object expands.

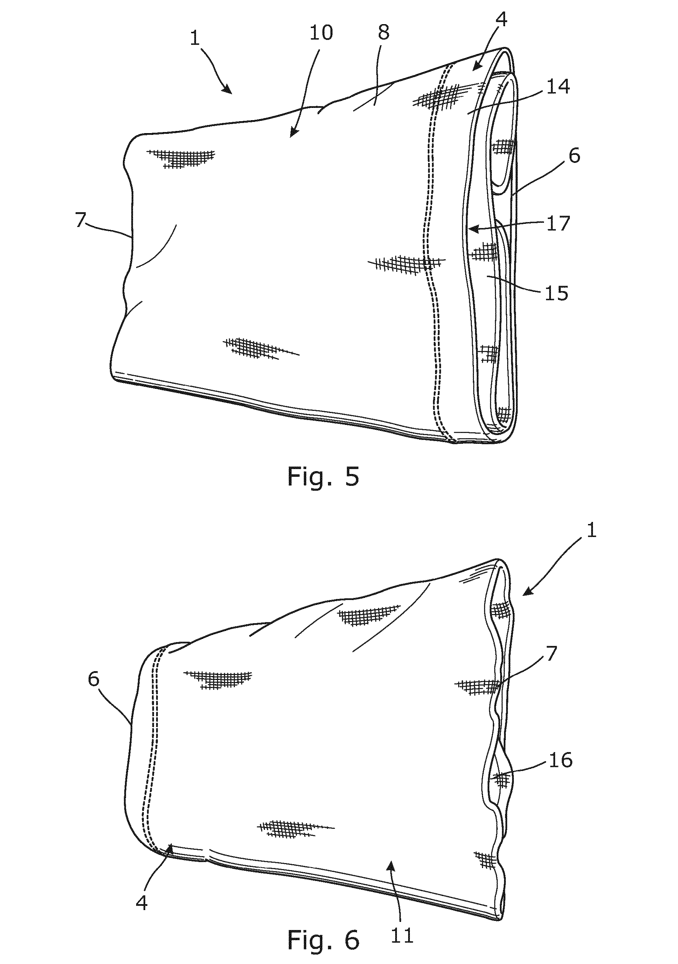

Figs. 5 to 8 show another embodiment of a fixation garment 1. In Fig. 5, the fixation garment 1 is shown from the first side 10 in the ready-to-use stage, i.e. with the second layer inserted into the first layer 8. From Fig. 5 the opening 17 between the first rib edge 14 and the second rib edge 15 is visible.

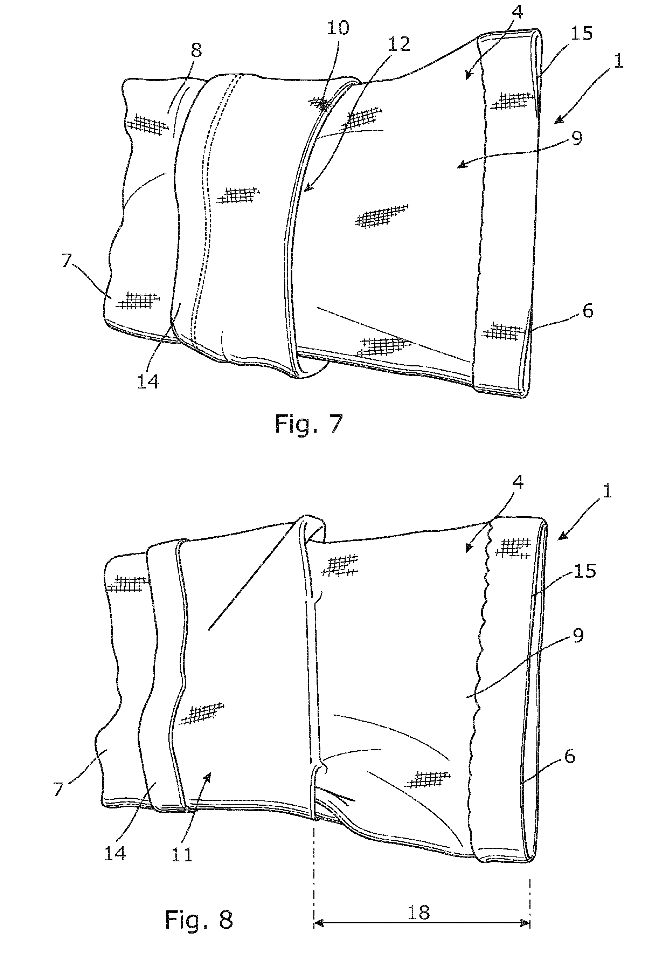

Fig. 6 shows the second side 11 and the fixation garment 1. In this embodiment, the aperture 16 is arranged at and near the second end 7. In Fig. 7, the first layer 8 is folded downwards towards the second end 7 so that easy access to the pocket 12 is facilitated. As described above, insertion of the object into the pocket 12 is facilitated since the object may be pushed into the pocket and the first layer 8 may then be folded back so that it substantially covers the object. By having the possibility of folding the first layer 8 downwards towards the second end 7, it is furthermore obtained that the aperture at the second end may be accessed from the inside of the pocket 12 so that for instance a discharge pipe from a urine bag may easily be led through the aperture.

Fig. 8 shows the opposite side 11 wherein the first layer is folded downwards towards the second end 7. The first layer is folded downwards to the area where the second layer 9 and the first layer are interknitted. The first and second layers are interknitted at a distance 18 from the first end 6.

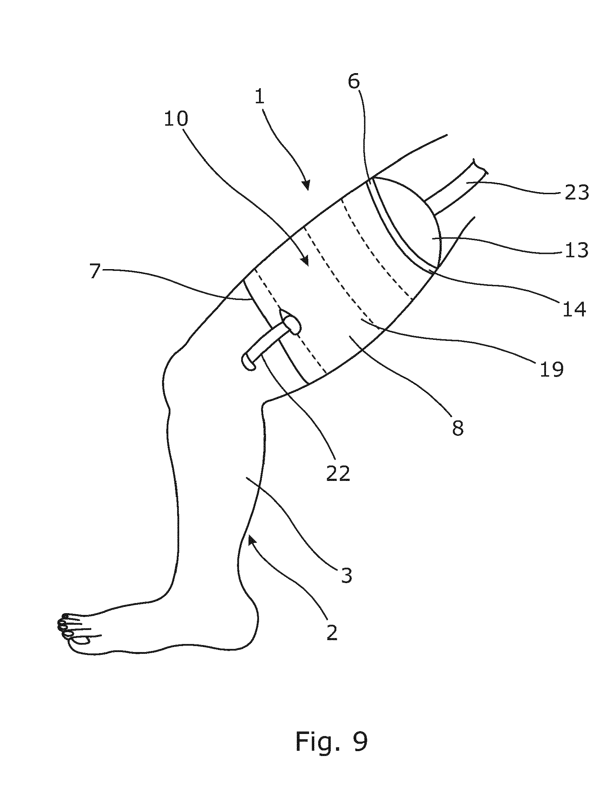

Fig. 9 shows a preferred use of the fixation garment 1 according to the invention. In this respect it is used as a fixation garment 1 for fixating a urine bag 13 to a leg 3 of a wearer 2. The urine bag 13 has an inlet 23 extending out of the pocket at the first end 6 and a discharge pipe 22 extending out of the aperture at the second end 7 of the fixation garment 1.

In other embodiments, the weft-knitted fixation garment may be used for instance for fixing an ice pack, an electrical device or similar objects to an arm or leg of a wearer.

Although the invention has been described in the above in connection with preferred embodiments of the invention, it will be evident for a person skilled in the art that several modifications are conceivable without departing from the invention as defined by the following claims.

## Claims

1. A method for providing a weft-knitted fixation garment (1) to be worn on a wearer's (2) arm or leg (3), the fixation garment (1) comprising a knitted tubular part (4) having a circumference less than 1000 mm, preferably less than 500 mm, the knitted tubular part (4) having a first end (6) and a second end (7) and comprising a first layer (8) and a second layer (9) extending therebetween, the weft-knitted fixation garment (1) comprising a first side (10) and a second side (11) and a pocket (12) adapted to fixate an object (13) to the wearer's arm or leg arranged at the first side (10), wherein the fixation garment (1) is integrally knitted in one process on a circular knitting machine by:
- knitting a first rib edge (14) of the tubular part (4),
- knitting the first layer (8) of the tubular part (4),
- knitting the second layer (9) of the tubular part (4) directly after the first layer (8),
- knitting a second rib edge (15) of the tubular part (4), and
- inserting the second layer (9) into the first layer (8) for providing a seamless weft-knitted fixation garment (1) ready for use.

2. A method according to claim 1, comprising the steps of:
- hanging up a plurality of stitches on transfer jacks or needles at a predetermined distance after the first rib edge (14), and
- knitting in the hanged-up plurality of stitches at a predetermined distance before a second rib edge (15) so that the first and second layers (8,9) of the tubular body (4) are interknitted.

3. A method according to claims 1 or 2, wherein an aperture (16) is provided at the second end (7) on the first side (10).

4. A method according to any of the preceding claims, wherein an opening (17) to the pocket is provided at the first end (6) between the first and second rib edges (14, 15).

5. A method according to claim 2, wherein the two layers (8, 9) are knitted together along the entire circumference or part of the circumference of the tubular part (4).

6. A method according to any of the preceding claims, wherein one or more fixation zones (19) are provided in the first layer (8), the fixation zones extending around the circumference of the first layer (8) and being positioned at mutual distances in a length direction of the first layer.

7. A method according to claim 3, wherein the aperture (16) is provided in the first layer (8) at a second distance from the second end (7).

8. A method according to any of the preceding claims, wherein an intermediate area (21) is knitted along the circumference between the first and second layers (8, 9) with an elasticity which is higher than that of the adjacent first and second layers.

9. A weft-knitted fixation garment (1) for fixing an object (13) to an arm or leg (3) of a wearer (2) provided by the method according to any of the preceding claims, the fixation garment (1) comprising a knitted tubular part (4) having a circumference less than 1000 mm, preferably less than 500 mm, the knitted tubular part (4) having a first end (6) and a second end (7) and comprising a first layer (8) and a second layer (9) extending therebetween, the weft-knitted fixation garment (1) comprising a first side (10) and a second side (11) and a pocket (12) adapted to fixate an object (13) to the wearer's arm or leg arranged at the first side (10).

10. A weft-knitted fixation garment (1) according to claim 9, wherein the two layers (8, 9) are knitted together at the second side (11) at a predetermined distance from the first end (6).

11. A weft-knitted fixation garment (1) according to any of the preceding claims 10-10, wherein the two layers (8, 9) are knitted together along the entire circumference or part of the circumference of the tubular part (4).

12. A weft-knitted fixation garment (1) according to any of the preceding claims 10-11, wherein the first layer (8) comprises one or more fixation zones (19), the fixation zones (19) extending around the circumference of the first layer (8) and being positioned at mutual distances in a length direction of the first layer (8).

13. A weft-knitted fixation garment (1) according to claim 9, wherein an aperture (16) is arranged in the first layer (8) at a second distance from the second end (7).

14. A weft-knitted fixation garment (1) according to any of the preceding claims 10-13, wherein the second end (7) has an intermediate area (21) extending along the circumference between the first and second layers (8, 9), the intermediate area (21) having a higher elasticity than the adjacent first and second layers.

15. Use of the weft-knitted fixation garment (1) according to any of the claims 10-14 for fixing a urine bag, an ice pack, an electrical device or any other objects to an arm or leg of a wearer.
